# EUROPEAN PATENT SPECIFICATION

(11) **EP 1 527 188 B1**
(45) Date of publication and mention of the grant of the patent: **27.01.2010**
(21) Application number: 03783975.0
(22) Date of filing: 11.06.2003
(51) Int. Cl.: C12P 21/02, C07K 14/535

(54) **PROCESS FOR THE PRODUCTION OF INCREASED AMOUNTS OF CORRECTLY FOLDED HETEROLOGOUS PROTEIN IN INCLUSION BODIES**
VERFAHREN ZUR HERSTELLUNG ERHÖHTER MENGEN AN RICHTIG GEFALTETEN HETEROLOGEN PROTEINEN IN EINSCHLUSSTEILCHEN
PROCEDE PERMETTANT D'AUGMENTER LES TAUX DE G-CSF CORRECTEMENT PLIES DANS DES CORPS D'INCLUSION

(30) Priority: 31.07.2002 SI 200200187
(43) Date of publication of application: 04.05.2005
(73) Proprietor: LEK Pharmaceuticals d.d., 1526 Ljubljana (SI)
(72) Inventor: MENART, Viktor, 1370 Logatec (SI); GABERC-POREKAR, Vladka, 1000 Ljubljana (SI); JEVSEVAR, Simona, 2000 Maribor (SI)
(74) Representative: Kröger, Bernd
(86) International application number: PCT/EP2003/006134
(87) International publication number: WO 2004/015124

(56) References cited:
- EP-A- 0 217 404
- EP-A- 0 913 469
- WO-A-99/18196
- WO-A-03/051922
- US-A- 5 912 327
- THOMAS JEFFREY G ET AL: "Divergent effects of chaperone overexpression and ethanol supplementation of inclusion body formation in recombinant Escherichia coli" PROTEIN EXPRESSION AND PURIFICATION, vol. 11, no. 3, December 1997 (1997-12), pages 289-296, XP002258354 ISSN: 1046-5928
- LU H S ET AL: "FOLDING AND OXIDATION OF RECOMBINANT HUMAN GRANULOCYTE COLONY STIMULATING FACTOR PRODUCED IN ESCHERICHIA-COLI CHARACTERIZATION OF THE DISULFIDE-REDUCED INTERMEDIATES AND CYSTEINE-SERINE ANALOGS" JOURNAL OF BIOLOGICAL CHEMISTRY, vol. 267, no. 13, 1992, pages 8770-8777, XP002258355 ISSN: 0021-9258
- CHALMERS J J ET AL: "EFFECTS OF TEMPERATURE ON ESCHERICHIA-COLI OVERPRODUCING BETA LACTAMASE OR HUMAN EPIDERMAL GROWTH FACTOR" APPLIED AND ENVIRONMENTAL MICROBIOLOGY, vol. 56, no. 1, 1990, pages 104-111, XP008023509 ISSN: 0099-2240

## Description

### Field of the invention

The present invention relates to a new process for the production of a heterologous protein from inclusion bodies which are formed in an organism in which the heterologous protein is expressed. The principle of the present invention can be applied to a wide range of heterologous proteins to be expressed from a suitable expression organism, although it will be mainly described by referring to the process for the production of biologically active granulocyte-colony stimulating factor (G-CSF).

### Background of the invention

Human granulocyte colony-stimulating factor (hG-CSF) belongs to hematopoetic growth factors which has a decisive role in the formation of neutrophils. G-CSF is used in medicine in the field of hematology and oncology. Today, two forms for clinical use are on the market: lenograstim which is glycosylated and is produced in mammalian cells, specifically a CHO cell line (Holloway CJ (1994) Eur J Cancer 30A Suppl 3:S2-S6., EP 169566), and filgrastim which is nonglycosylated and is produced following its expression in a bacterium *E. coli* (EP 237545).

For intracellular production of heterologous G-CSF in the bacterium *E. coli,* the protein is accumulated in the form of inclusion bodies (classical inclusion bodies). In experiments of secretion into *E. coli* periplasm, G-CSF is accumulated either in the form of classical inclusion bodies (Lei S.P et al (1987) J Bacteriol 169:4379-4383; Chung BH et al (1998) J Ferment Bioeng 85:443-446) or there has been no report on biological activity of G-CSF produced in this way (Jeong KJ & Lee SY (2001) Protein Expression and Purification 23: 311-318).

From the aforementioned, it is clear that in almost all described experiments of isolation of G-CSF from the bacterium *E. coli* in the prior art, G-CSF is found in classical inclusion bodies.

Similar observations on the formation of classical inclusion bodies also apply to the production process of heterologous protein other than G-CSF.

Several examples of classical inclusion bodies produced in yeast have been described, however, there has been no report on G-CSF.

For production of correctly folded biologically active proteins from inclusion bodies which are insoluble in water based liquids under native conditions (classical inclusion bodies), the inclusion bodies should be isolated from the cells, washed and solubilised, and then *in vitro* renaturation should be performed. *In vitro* renaturation is an additional step in the process of large-scale isolation of proteins.

Processes for the production of recombinant proteins from classical inclusion bodies comprise lysis and disruption of the cells followed by centrifuging. The pellet comprising a large proportion of classical inclusion bodies is usually washed with detergents. For the isolation of G-CSF, washing of classical inclusion bodies with 1 % deoxycholate (Zsebo KM et al (1986) Immunobiology 172:175-184. EP237545; US5849883) or with a solution of 1.0 M Nacl with added 0.1 % Tween 80 (Gelunaite L et al (2000) J Chromatogr A 904:131-143) has been reported to be convenient.

A further step in obtaining recombinant proteins is the solubilisation of classical inclusion bodies requiring generally the use of rather strong denaturants. Referring to the production of G-CSF, the use of 6M GdHCl (Wingfield P et al (1988) Biochem J 256:213-218; Zink T et al (1992) FEBS Lett 314:435-439.), 7 M urea at pH 7.2 (Kuga T et al (1989) Biochem Biophys Res Commun 159:103-111) or 8 M urea pH 8.0 (Yamasaki M et al (1998) Biosci Biotechnol Biochem 62:1528-1534), 20 mM Tris/HCl and the addition of EDTA and DTT has been described. Kang S-H and co-workers succeeded to solubilise classical inclusion bodies in 2 M urea in strongly alkaline pH about 12 (Kang SH et al (1995) Biotechnol Lett 17:687-692). A similar process is the solubilisation in 10 mM HEPES buffer with a short-term increase of pH to 11.5 (Gelunaite L et al z(2000) J Chromatogr A 904:131-143). Strong denaturants in denaturating concentrations are equally effective for solubilisation, for example, 1% SDS with added 5% ethanol in 50 mM Tris/HCl pH 8.5 (Souza LM et al (1986) Science 232:61-65). 2% N-lauroyl sarcosine is often used (Lu HS et al (1992) J Biol Chem 267:8770-8777; Lu HS et al (1993) Protein Expr Purif 4:465-472; Young DC et al (1997) Protein Science 6: 1228-1236; EP237545; US5849883) or 1% N-lauroyl sarcosine alone (Zsebo KM et al (1986) Immunobiology 172:175-184). In most cases, solubilisation of classical inclusion bodies requires the use of strong detergents which are toxic and non-nature-friendly and are serious environmental pollutants. Their use is also uneconomical, because safe removal after the end of the process is an additional cost and is time-consuming.

Although some examples of the separation of protein under denaturating conditions in the first chromatographic step, for example on C4 RP HPLC (Souza LM et al 1986) Science 232:61-65; Zsebo KM et al (1986) Immunobiology 172:175-184; EP237545) or by using gel filtration (Wingfield P et al (1988) Biochem J 256:213-218; US4999291) are described, in the majority of examples the chromatographic separation runs after preliminary *in vitro* renaturation.

The solubilisation of classical inclusion bodies in strong denaturants is followed by *in vitro* renaturation of proteins. Referring again to the exemplified G-CSF, its *in vitro* renaturation is achieved by dialysis against the buffer with a lower (non-denaturating) concentrationof a denaturant (Wingfield P et al (1988) Biochem J 256:213-218), by dialysis against the buffer without a denaturant (Kuga T et al (1989) Biochem Biophys Res Commun 159:103-111*),* in the buffer with added 0.8 M arginine (Zink T et al (1992) FEBS Left 314:435-439) or by gel filtration (US4999291; D.C. Young et al (1997) Protein Science 6: 1228-1236). After solubilisation in denaturing concentrations of N-lauroyl sarcosine, oxidative folding is achieved with the addition of CuSO₄ in low concentrations during long-term stirring at room temperature, followed by removal of a detergent by using a strong base ion exchanger (US5849883). In cases where the solubilisation runs under extremely alkaline conditions, the protein is spontaneously renaturated by restoring a lower pH (Kang SH et al (1995) Biotechnol Lett 17:687-692). All described methods for *in vitro* renaturation are complex and time-consuming. Large-scale dilution requires a large capacity apparatus and consequently a higher initial investment (larger space, larger apparatus, etc.), and for the production higher operating costs (power, water, buffers, etc.).

Improved *in vitro* renaturation is achieved in fusion proteins wherein an oligopeptide hydrophilic tag and a restriction site for IgA protease are added to the N-terminus (EP0500108).

In the literature an increase in proportion of soluble protein in the cytoplasm has been reported (Weickert MJ et al (1997), Appl and Environmental Microbiology, 63:4313-4320; Bhandari P & Gowrishankar J (1997) J. Bacteriol, 179: 4403-4406; Zhang Y et al (1998) Protein Expr Purif, 12: 159-165; Thomas JG and Baneyx F (1996) J of Biological Chemistry, 271:11141-11147; Kapust RB and Waugh DS (1999) Protein Science, 8: 1668-1674; Davis GD et al (1999) Biotech and Bioeng 65: 382-388), but there has been no eport on the production of soluble G-SCF in the cytoplasm instead of its accumulation ininclusion bodies.

To our knowledge, there has so far been no reports in the patent and scientific literature on the increase of the proportion of correctly folded heterologous proteins already in the inclusion bodies.

In the case of G-CSF, *in vitro* renaturation is reported as the first condition and thus the only possible way for the production of biologically active protein (Rudolph R (1996) in Protein Engineering: Principles and Practice (Cleland JL and Craik CS eds) pp 283-298, Wley-Liss, Inc., New York).

### Summary of the invention

It is an object of the invention to provide an improved process for the production of a heterologous protein, which involves obtaining the heterologous protein from non-classical inclusion bodies which are formed in the organism in which the heterologous protein is expressed.

The object is solved by any one of the alternative processes as defined in claims 1, 2, 27 and 33. Preferred and advantageous further developments of the processes according to the present invention are set forth in the dependent claims. The present invention also provides the use according to claim 36.

The concept of the present invention can be applied to a wide variety of heterologous proteins which can be expressed in suitable host cell systems such as microorganisms like bacteria and yeasts. The present invention was particularly developed on the production of G-CSF. Since the concept of the invention *inter alia* involves the formation of inclusion bodies which are formed from relatively hydrophobic proteins, the present invention can be readily applied to the production of other heterologous proteins as well, especially those which do not have too many disulphide bonds. The present invention is thus particularly suitable for the production of heterologous proteins of the group consisting of G-CSF, GM-CSF, M-CSF, EGF, HSA, DNAse, FGF, TNF-alpha, TNF-beta, interferons and interleukins.

As the basis for the processes according to the present invention, it was surprisingly found that G-CSF can be accumulated in the form of correctly folded precursors in inclusion bodies having an amount of the correctly folded precursor, higher than the classical inclusion bodies. Alternatively, the adjustment of the biosynthesis of G-CSF enables to produce, directly in the inclusion bodies, a substantial proportion of precursors of the heterologous protein which can form, under non-denaturating conditions or without a denaturation/renaturation step, the biologically active G-CSF. Also in the case of other heterologous proteins such as those mentioned above, it is expected that the accumulation of such other heterologous proteins in inclusion bodies is also feasible in the processes according to the present invention.

In one particular aspect of the invention, already the conditions or parameters of the biosynthesis, i.e. during the fermentation and protein expression process, are adjusted in order to control, and advantageously to promote or enhance, the formation of correctly folded precursors of heterologous proteins. It is assumed that a process which takes account of the way of performing the biosynthesis enables the accumulation of the correctly folded precursor of G-CSF, i.e. the heterologous protein, already in the inclusion bodies. Such a way of performing the process, i.e. adjusting and beneficially controlling the biosynthesis in terms of forming and increasing correctly folded precursors of the heterologous protein in the inclusion bodies, involves one or more conditions selected from the group comprising: cultivation temperature, composition of cultivation medium, induction mode, principle of performing the fermentation, addition of agents which are capable of causing stress, and co-expression of auxiliary proteins. The control of the biosynthesis comprises adjustment and/or changes when compared to conventional biosynthesis conditions, such as lower temperature, selecting fermentation medium and type of fermentation, selecting inducer and mode of induction, and inducing stress.

In another particular aspect of the invention, the precursors of the heterologous protein found in the inclusion bodies are kept, during the process of isolation and purification of the heterologous protein from the inclusion bodies, under conditions which are non-denaturating for the heterologous protein. A particularly preferred embodiment of the process for the production of biologically active heterologous protein of the present invention accordingly further comprises the solubilisation of inclusion bodies, which is preferably preceded by a washing step, under non-denaturating and preferably native conditions and enables the direct isolation of biologically active proteins, without the need of using denaturants or applying a denaturation/renaturation process. Since the present invention enables the production of inclusion bodies having a substantial proportion of correctly folded precursor of the heterologous protein (non-classical inclusion bodies), this particular aspect of the invention provides a very efficient way to the production of the protein, without a denaturation/renaturation step being required.

### Brief description of the drawings

Figure 1 shows an analysis, by means of SDS-PAGE and protein staining, of the protein composition in inclusion bodies obtained dependent on various conditions in accordance with the present invention;
Figure 2 shows, as analysed by means of SDS-PAGE and protein staining, a comparison of the solubilisation of the inclusion bodies depending on the cultivation temperature (25°C or 37°C) in accordance with the present invention;
Figure 3 shows an analysis, by means of SDS-PAGE and protein staining, of the composition of proteins in inclusion bodies depending on various conditions in accordance with the present invention; and
Figure 4 shows, as analysed by means of SDS-PAGE and protein staining, a comparison of the solubilisation of inclusion bodies depending on the washing/solubilisation with different solutions in accordance with the present invention.

### Description of the invention

The present invention will be described in the following in further detail with respect to both the basic concept and the preferred embodiments of the invention. The present invention is however not limited to the specifically described embodiments, and the skilled person will understand that variations and modifications exist within the scope of the annexed claims.

It has been surprisingly found that by affecting the cultivation or fermentation of the organism that expresses the heterologous protein, especially by affecting the biosynthesis performed by the organism, the production of biologically active G-CSF, and correspondingly that of other heterologous proteins, can be performed in such a way that a correctly folded precursor of G-CSF is accumulated in the inclusion bodies. Non-classical inclusion bodies can be formed in this manner.

Although the inclusion bodies described in the patent and scientific literature present insoluble compact aggregates of incorrectly folded or partially correctly folded proteins (classical inclusion bodies), in the present invention it has been achieved that the correctly folded precursor of G-CSF is accumulated in inclusion bodies and non-classical inclusion bodies can be formed. By observing more than one, preferably a combination of multiple conditions which will be described below and which beneficially affect the biosynthesis, the amount of correctly folded precursors of G-CSF found in inclusion bodies is remarkably increased. The inclusion bodies which comprise the correctly folded precursor of G-CSF (non-classical inclusion bodies) are more soluble than the inclusion bodies (classical inclusion bodies) found in the art. Higher solubility enables easier and less time-consuming production of G-CSF from inclusion bodies. Therefore, the yields are high, being useful, practical and economical in the large-scale production.

The term 'classical inclusion bodies' as used herein, refers to the insoluble or slightly soluble *(in media under non-denaturating conditions such as non-denaturating aqueous solutions)* inclusion bodies containing predominantly misfolded proteins which are described in the prior art.

The term 'non-classical inclusion bodies' as used herein, refers to the inclusion bodies which are more soluble *(in media under non-denaturating conditions such as non-denaturating aqueous solutions)* than the classical inclusion bodies and which comprise a certain amount of correctly folded precursor of a heterologous protein.

The term 'correctly folded precursor' of the heterologous protein (such as G-CSF), as used herein, refers to a protein (e.g. G-CSF) having the correct conformation (the native conformation may be present or inherently generated), wherein the disulphide bridges may not be formed yet or not be formed completely. Alternatively, the term 'correctly folded precursor' can be ascribed to a form of the heterologous protein which is present in the inclusion bodies, which form is capable of being directly solubilised without denaturants or strong alkaline solutions and is capable of providing a biologically active protein, This is demonstrated in the examples which refer to the exemplified heterologous protein G-CSF.

We suppose that the correctly folded precursor of G-CSF is somehow trapped within insoluble aggregates which are present in non-classical inclusion bodies, therefore, only isolation from these aggregates is required. The isolation is performed by the solubilisation of inclusion bodies, which solubilisation is preferably preceded by a washing step. For such isolation only mild solubilising agents should be used, and strong denaturating agents conventionally used for solubilisation of classical inclusion bodies can be avoided. The preceding washing step should be preferably performed under conditions that the correctly folded precursor is not dissolved by the washing solution. It is assumed that the process of isolation of G-CSF precursor enables the spontaneous oxidation (for example due to presence of air oxygen) and allows the spontaneous formation of the intramolecular disulphide bridges. If desired, the formation of disulphide bonds can be promoted by the treatment, suitable during washing and/or solubilisation, with small amount of catalyst such as CuSO₄ or some other metallic salts. The biologically active G-CSF is thereby obtained and can be directly isolated and purified by suitable purification techniques.

In comparison with other known procedures of production of heterologous proteins which, after expression, are found in classical inclusion bodies, the procedure of the present invention is better from the economical point of view. Instead of washing with strong detergents only washing with water or buffer is needed and the solubilisation runs under mild conditions and not in the presence of strong denaturants, strongly alkaline solutions, or detergents in high concentrations. Furthermore, there is no need for *in vitro* renaturation procedures normally required for refolding.

In a particular preferred embodiment of the present invention, in which the formation and accumulation of the correctly folded precursor of the heterologous protein (specifically of G-CSF) already in the inclusion bodies is particularly effective and the biologically active protein (e.g. G-CSF) can be obtained directly from the inclusion bodies, the process for production of biologically active protein (e.g. G-CSF) comprises adjusting the way of performing the biosynthesis by means of one or a combination of several parameters or conditions which are selected from the group consisting of:
- cultivation temperature,
- induction mode,
- type of fermentation,
- selection of cultivation medium
- adding agents that are capable of causing stress, and
- co-expression of auxiliary proteins.

The adjustment of these parameters can be performed in the way that the non-classical inclusion bodies are formed.

The process for production of biologically active heterologous protein (such as G-CSF) of the present invention preferably further comprises washing and/or solubilisation of inclusion bodies and enables the isolation of biologically active heterologous protein (such as G-CSF) without use of denaturants or *in vitro* renaturation process. Thus, the process for the production of the heterologous protein and the subsequent isolation and/or purification of the heterologous protein can be advantageously performed under native conditions all along the process, which is in favour of yield and provides a process which is both economical and environment-friendly.

A feature of the process of the present invention is that the way of performing the biosynthesis comprises adjusting at least one of the parameters or conditions which enable the regulation of the composition of inclusion bodies in such a way that the proportion of the correctly folded protein molecules (especially those of G-CSF, notably the precursors of G-CSF) is increased. High accumulation of the heterologous protein does not automatically mean a high proportion of the correctly folded precursor of the heterologous protein. The process for production of biologically active heterologous protein of the present invention also enables to find an optimal ratio between accumulation of the heterologous protein and the proportion of correctly folded precursor of the heterologous protein (especially in case of G-CSF as the heterologous protein).

A high yield large-scale production of biologically active G-CSF is thereby attained and expected also for other heterologous proteins.

The term 'biologically active G- CSF' used herein refers to G-CSF which is capable of promoting the differentiation and proliferation of hematopoietic precurser cells and the activation of mature cells of the hematopoietic system. With the process of the present invention, it is possible to obtain a specific activity of G-CSF which beneficially lies in a range of at least 1x10⁷ IU/mg, more preferably in a range of specific activity above 4x10⁷ IU/mg. When applying a suitable subsequent purification process, the specific activity can be increased to a range of at least 7-8x10⁷ IU/mg, most preferably to a range of specific activity of at least 1x10⁸ IU/mg. The specific activity is measured by a method based on stimulation of cellular proliferation as described in example 12.

The term 'heterologous protein', as used herein, refers to the protein which is foreign to the organism in which expression is performed.

The term 'renaturation', as used herein, refers to the conversion of denaturated proteins to a conformation which the proteins have in their native conformations.

The term '*in vitro* renaturation', as used herein, refers to the renaturation which is performed outside the organism.

The term 'denaturation', as used herein, refers to a process in which the native conformation of the protein (three-dimensional structure) is changed but the primary structure (amino acid chain, peptide links) of the protein remains unchanged.

The term 'denaturant', as used herein, refers to an agent, in the presence of which (normally in solution) the native conformation of proteins is not preserved. Biological activity of the proteins in the presence of denaturants is changed and is not preserved.

The term 'aggregate', as used herein, refers to the cluster of the molecules which are mutually linked predominantly with hydrophobic as well as with other bonds (as for example disulphide bonds). These molecules are not biologically active.

The term 'non-denaturating conditions' used herein refers to conditions such that denaturation of the desired protein does not occur. This conditions refer to e.g., conditions where no denaturant is present or is present below denaturing concentrations.

The term 'native conditions' used herein refers to conditions in which the protein molecule can preserve the native conformation and the biological activity.

The term 'biosynthesis', as used herein, refers to the production of a heterologous protein by using microorganisms.

The term 'cultivation', as used herein, refers to the growth of microorganisms under controlled conditions, wherein the microorganisms are submersed, or on solid supports with the provided source of substrates needed for the growth of microorganisms.

The term 'fermentation', as used herein, refers to cultivation of microorganisms under submersed conditions in a bioreactor (fermentor) or shake flasks.

The terms 'accumulation of heterologous protein' and 'accumulation of G-CSF', as used herein, refers to a proportion of heterologous protein and G-CSF, respectively obtained by using the heterologous expression of the gene for the heterologous protein or G-CSF, with regard to total proteins. If desired, it is feasible according to the present invention to accumulate the desired, correctly folded heterologous protein produced in the expression system to a proportion of at least about 20%, more preferably at least about 25% and particularly at least about 30% and higher, relative to the total protein mass of the host cell used in the expression system. The term 'heterologous expression', as used herein, refers to the expression of those genes which are foreign to the organism in which the expression is performed.

The term 'proportion of correctly folded precursor of heterologous protein' (such as G-CSF), as used herein, refers to the proportion of the correctly folded precursor of heterologous protein (e.g. G-CSF) with respect to the total of the heterologous protein forms (e.g. G-CSF proteins), i.e. including correctly, partly correctly, incorrectly folded forms.

The term 'proportion of biologically active heterologous protein' (such as G-CSF), as used herein, refers to the proportion of the correctly folded precursor of heterologous protein (e.g. G-CSF) after washing and solubilisation of (non-classical) inclusion bodies. During the process of the invention, especially during and after the washing and/or solubilisation, the correctly folded precursor of the heterologous protein (e.g. G-CSF) can be rendered biologically active due to spontaneous oxidation and, hence, spontaneous formation of the disulphide bonds.

The process for production of biologically active heterologous protein such as G-CSF of the present invention enables the preparation of the heterologous protein such as G-CSF which is suitable for clinical use in human and veterinary medicine.

It can be used for the preparation of human G-CSF and other mammalian G-CSF, as well as for the preparation of G-CSF derivatives, such as methionyl G-CSF (Met-G-CSF), enzymatically and chemically modified (such as, for example pegylated, succinylated) G-CSF, G-CSF analogs and fusion proteins which comprise G-CSF.

While the above description of the present invention was particularly referred to the production of biologically active G-CSF, the process of the present invention can be also used for the production of other biologically active heterologous proteins to be accumulated in the form of non-classical inclusion bodies. These proteins are selected from the group comprising: interferons (IFN), such as INF-beta 1b, IFN-beta 2b, IFN-gamma 1b, interleukins (IL), such as IL-2 and IL-4, granulocyte macrophage-colony stimulating factor (GM-CSF), macrophage-colony stimulating factor, (M-CSF), epidermal growth factor (EGF), human serum albumin (HSA), deoxyribonuclease (DNAse), fibroblast growth factor (FGF), tumour necrosis factor alpha (TNF alpha) and tumour necrosis factor beta (TNF-beta) and further comprises other proteins, including fusion proteins, with not too many disulphide bonds which (at least in some and preferably in a substantial proportion) can be spontaneously folded into the correct 3D structure.

The process for production of a biologically active protein such as G-CSF of the present invention can be used in the case of formation of the non-classical inclusion bodies irrespective of the organism used as the host for expression. As suitable host organisms, there can be used those selected from the group comprising primarily bacteria and yeasts. Of bacterial systems *E*. *coli* and *Streptomyces* spp., and of yeast systems conventional yeast strains such as *Saccharomyces cerevisiae,* and unconventional yeast strains such as *Pichia pastoris, Hansenula polymorpha, Candida utilis* and others can be employed.

The way of performing the biosynthesis of the heterologous protein such as G-CSF of the present invention comprises adjusting the conditions or parameters which are selected from the group: cultivation temperature, composition of the cultivation medium, induction mode, principles or type of performing the fermentation, addition of agents causing stress, and co-expression of auxiliary proteins. By optimising an individual parameter of the aforementioned parameters, it is possible to increase substantially the proportion of the correctly folded precursor of the heterologous protein, as found with G-CSF, and the combination of the parameters enables additionally higher proportion. The conditions or parameters will be described in more detail in the following while referring to the preferred embodiment of producing G-CSF by biosysthesis.

### Cultivation temperature

It has been surprisingly found that decreasing the cultivation temperature enables the accumulation of a correctly folded precursor of G-CSF already in non-classical inclusion bodies. Normal optimal cultivation temperature of e.g. *E. coli* cells is 37°C. In the present invention, it has been found that the preferable temperature for the accumulation of the correctly folded precursor of G-CSF already in non-classical inclusion bodies is significantly lower than 37°C, namely, between about 20°C and about 30°C. The most preferable temperature is about 25°C.

### Induction mode

It has been surprisingly found that the proportion of the correctly folded precursor of G-CSF in inclusion bodies (non-classical) also depends on the induction mode. Induction is possible with the addition of an inducer which is preferably selected from the group consisting of IPTG, lactose and NaCl. Particularly preferred induction is with IPTG. The proportion of the correctly folded precursor of G-CSF in non-classical inclusion bodies also depends on the concentration of the inducer. When IPTG is added the chosen concentration is preferably in the range from about 0.1 mM to about 1 mM. The more preferred concentration is about 0.3 to 0.6 mM, particularly about 0.4 mM. When NaCl is used the chosen concentration is preferably in the range from 0.3 M to 1.3 M. The more preferred concentration is about 1.0 to 1.3 mM, particularly about 1.2 M. When lactose is used the chosen concentration is preferably in the range between about 1 and about 10 g/l, and the most preferred concentration is between about 2 and about 4 g/l. The proportion of the correctly folded precursor of G-CSF in inclusion bodies also depends on the induction mode (the time of cultivation). The inducer can be added at the beginning of fermentation (while when a preculturing step is performed: instantaneously subsequent to that preculturing step), which is preferable in case of IPTG, NaCl or lactose. IPTG and NaCl can be also added in later steps of the fermentation, suitably at an OD₆₀₀ₙₘ of about 6.0 (OD is the measure for the bacterial count).

### Principle or type of performing the fermentation

It has been surprisingly found that proportion of the correctly folded precursor of G-CSF in non-classical inclusion bodies also depends on the type of performing the fermentation. It is selected from the group comprising: fermentation in a bioreactor and performing the fermention in shake flasks. Performing the fermentation in a bioreactor is preferable which comprises performing the fermentation in a batch mode and performing the fermentation in a fed-batch mode. The preferred principle or type of performing the fermentation is a batch mode where the high productivity of biomass with highly soluble inclusion bodies is obtained.

### Co-expression of auxiliary proteins or addition of stress-causing agents

Further it has been found that the proportion of the correctly folded precursor of G-CSF in inclusion bodies also depends on the addition of agents capable of causing stress. These additives trigger co-expression of stress proteins and are preferably selected from the group comprising ethanol and propanol, which agents can be used in various concentrations in the range from 1% to 5% (v/v). Use of ethanol and propanol at a concentration of about 3% (v/v) is most preferable.

### Composition of cultivation medium

Further it has been surprisingly found that the proportion of the correctly folded precursor of G-CSF in non-classical inclusion bodies also depends on the composition of the cultivation medium. Preferred media are selected from the group comprising: GYST (20 g/l tryptone, 5 g/l yeast extract, 10 g/l NaCl, 10 g/l glucose, metals in traces) supplemented with antibiotic, e.g. 100 mg/l ampicillin GYSP (20 g/l phytone, 5 g/l yeast extract, 10 g/l NaCl, 10 g/l glucose, metals in traces) supplemented with antibiotic, e.g. 100 mg/l ampicillin, and LYSP (20 g/l phytone, 5 g/l yeast extract, 10 g/l NaCl, 6 g/l glycerol, 2 g/l or 4 g/l lactose, metals in traces) supplemented with antibiotic, e.g. 100 mg/l ampicillin, LYST (20 g/l tryptone, 5 g/l yeast extract, 10 g/l NaCl, 6 g/l glycerol, 2 g/l or 4 g/l lactose, metals in traces) supplemented with antibiotic, e.g. 100 mg/l ampicillin), LBON (10 g/l phytone, 5 g/l yeast extract) supplemented with antibiotic, e.g. 100 mg/ml ampicillin, GYSPON (20 g/l phytone, 5 g/l yeast extract, 10 g/l glucose, metals in traces) supplemented with antibiotic, e.g. 100 mg/l ampicillin (Metals in traces: (FeSO₄x7H₂O: 40 mg/l, CaCl₂x2H₂O: 40 mg/l, MnSO₄xnH₂O: 10 mg/l, AlCl₃x6H₂O: 10 mg/l, CoCl₂x6H₂O: 4 mg/l, ZnSO₄x7H₂O: 2 mg/l, NaMoO₄x2H₂O: 2 mg/l, CuSO₄x5H₂O: 1 mg/l, H₃BO₃: 0.5 mg/l)). Preferably, the GYST and GYSP media supplemented with 100 mg/ml ampicillin are used.

### Washing of inclusion bodies

A higher proportion of the biologically active protein in inclusion bodies leads to a higher solubility of inclusion bodies. If the inclusion bodies were washed with detergents, high proportion of correctly folded precursor of G-CSF would be washed away with the washing solution. Therefore, washing is preferably performed with a washing solution which is selected from the group consisting of water and various buffers in very low concentrations (1mM to 10mM), for example, buffer Tris/HCl, phosphate buffer, acetate buffer, citrate buffer. Most preferred is washing with water. The washing solution is preferably adjusted to a low temperature, suitable around 4°C. The washing can be performed once or multiple times.

### Solubilisation of inclusion bodies

The higher solubility of non-classical inclusion bodies which occurs due to a higher solubility of a correctly folded precursor of heterologous protein in the inclusion bodies indicates that the solubilisation can be advantageously performed under mild conditions, without the addition of strong denaturants, strongly alkaline solutions or denaturating concentrations of detergents. For the solubilisation of the inclusion bodies, the solvents to be used can be selected from the group consisting of: urea in non-denaturing concentrations (1-2 M, preferably in a buffer at a pH of below 10 and more preferably at a pH of about 8.0), N-lauroyl sarcosine in non-denaturing concentrations (0.05-0.25% (m/v)), low concentrations of Zwittergents, different non-detergent sulfobetains (NDSB), betain, sarcosine, carbamoyl sarcosine, taurine, dimethylsulfoxide (DMSO) and higher concentrations of buffers (preferably at a pH being maintained at below 10 and more preferably about 8.0), for example: HEPES, HEPPS, MES, ACES, MES. Preferably N-lauroyl sarcosine, NDSB and DMSO are used. Most preferably N-lauroyl sarcosine in the concentration range 0.1% to 0.25% (m/v) is used.

Cultivation temperature of about 20-30°C, specifically about 25°C; performing of fermentation in a batch mode; cultivation medium GYST or GYSP, each supplemented with antibiotic, e.g. 100 mg/ml ampicillin; induction mode by adding, in case of an optional preculturing step after said preculturing step, at the beginning of the fermentation, IPTG with a concentration of about 0.3 to 0.6 mM, preferably about 0.4 mM; optionally washing with water and solubilising inclusion bodies in N-lauroyl sarcosine at the concentration of about 0.1 to 0.25%, preferably about 0.2%, are optimised parameters where the proportion of correctly folded precursor of G-CSF (and consequently the proportion of biologically active G-CSF) and the accumulation of G-CSF are particularly high. With these parameters, the composition of the resulting proteins enables the G-CSF obtained in this manner to be used directly in further isolation procedures, such as extraction, conventional or expanded-bed chromatographic steps, for example ion-exchange, hydrophobic-interaction, size-exclusion or affinity, and preferably as the loading solution for immobilised metal affinity chromatography - IMAC. This process can be used in the large-scale production of G-CSF with high yields.

As described above, the formation of the biologically active heterologous protein can be attained by the preferred embodiment of the process of the invention, wherein no treament for denaturation and/or renaturation is required, and wherein the whole process including the isolation and purification of the desired heterologous protein is performed under non-denaturating and preferably under native conditions. Suitable techniques for the isolation and/or purification of the desired heterologous protein are known to the person skilled in the art and can be used, e.g., classical or expanded-bed chromatography using any of well known principles, e.g., ion-exchange, hydrophobic-interaction, affinity or size-exclusion, as well as continuous and batch-mode extractions using appropriate matrices or solutions The preferred technique is immobilised metal affinity chromatography (IMAC), as it enables a highly efficient preparation of pure and biologically active protein in high yield and under native conditions.

### Examples

### Example1: Influence of the cultivation temperature on the proportion of the correctly folded precursor of G-CSF in inclusion bodies and on the concentration of total proteins after solubilisation of the inclusion bodies

Human gene for G-CSF was modified for high expression in a bacterium *E. coli* (Fopt5). In the expression system *E. coli* BL21 (DE3) with pasmid pET3a, the level of accumulation of G-CSF is achieved accounting for over 50% of all cell proteins. *E. coli* culture BL21 (DE3) pET3a/P-Fopt5 from the strain bank at -70°C was inoculated at a ratio 1 to100 in LBG medium (10 g/l tryptone, 5g/l yeast extract, 10 g/l NaCl, 2.5 g/l glucose), supplemented with 100 mg/l ampicillin) and the culture was cultivated for 10 hours on a shaker at 25°C, 160 rpm. Two to 4 ml of this culture was inoculated in 40 ml GYST medium supplemented with 100 mg/ml ampicillin to which the IPTG inducer to the final concentration of 0.4 mM was added at the beginning of the fermentation (initial OD_{λ600nm} of the culture was 0.385). The culture was then cultivated on a shaker (shake flasks) at three different temperatures and at 160 rpm to the exponential phase of growth:
- at 25°C: 22 hours, final OD_{λ600nm} of the culture was 15.2 ± 0.5;
- at 37°C: 15 hours, final OD_{λ600nm} of the culture was 12.2 ± 0.4;
- at 42°C: 15 hours, final OD_{λ600nm} of the culture was 6.75 ± 0.2.

After completed cultivation, the resulting cultures were centrifuged for 10 minutes at 5000 rpm, washed with a 4-5-fold volume of 10 mM TrisHCl/pH=8.0 and the biomass was transferred into the new centrifuge tubes and centrifuged again for 10 minutes at 5000 rpm. The biomass was resuspended in a 5-fold volume of 10 mM TrisHCl/pH=8.0 buffer and sonicated 12 x for 1 minute with a submersed probe (duty cycle: 60%, power: 7, pulses: 2 s⁻¹). After sonification the broken cells were centrifuged again for 30 minutes at 5000 rpm. The pellet with inclusion bodies was solubilised under non-denaturating conditions in a 50-fold volume of 0.2 % N-lauroyl sarcosine in 40 mM TrisHCl/pH=8.0, solubilisation was left to run overnight 16 to 18 hours at room temperature, shaking at 50 rpm. After solubilisation, the protein concentration was determined according to the Bradford method using pure hMet-G-CSF as a standard. The concentrations were about 1 mg/ml for 42°C and 37°C and between 2 and 3 mg/ml for 25°C. The total protein concentrations after solubilisation of theinclusion bodies in 0.2% N-lauroyl sarcosine and the proportion of correctly folded precursor of G-CSF and biologically active G-CSF, respectively, prepared from the biomass cultivated at different temperatures are shown in Table 1. The proportion of the correctly folded precursor of G-CSF and biologically active G-CSF, respectively, (after extraction from the inclusion bodies) was determined by measuring the biologically activity of the solubilised inclusion bodies without removal of N-lauroyl sarcosine (at this dilution N-lauroyl sarcosine does not interfere with the biological activity measurement).

**Table 1**

| Cultivation temperature | Concentration of total proteins after solubilisation of inclusion bodies in 0.2 % N-lauroyl sarcosine | Proportion of biologically active G-CSF |
|---|---|---|
| 25°C | 2.7 mg/ml | > 30% |
| 37°C | 1.1 mg/ml | > 20% |
| 42°C | 1.0 mg/ml | < 1% |

As evident from the data presented in Table 1, the proportion of the correctly folded precursor of G-CSF in the inclusion bodies was markedly increased with cultivation at decreased temperature. Further, up to 2.5-fold higher concentration of total proteins were obtained after solubilisation of the inclusion bodies in 0.2 % N-lauroyl sarcosine suggesting that the inclusion bodies prepared this way are essentially more soluble. Non-classical inclusion bodies thus have been formed.

### Example 1: Influence of the induction mode with IPTG on the accumulation of G-CSF.

### Induction with IPTG at the beginning of the fermentation

Previous experiments with different concentrations of IPTG (0.05 mM - 0.4 mM) showed that a sufficiently high and almost the same accumulation of G-CSF was achieved in the range 0.1- 0.4 mM for the biomass with the final OD_{λ600nm} = 20-30. *E. coli* BL21(DE3) pET3a/P-Fopt5 from the strain bank at -70°C was inoculated in a ratio 1 to 500 in the LBG/amp medium and cultivated for 14-18 hours on a shaker at 25°C, 150 rpm. This culture was used as an inoculation culture to inoculate the fermentor culture in a ratio 1 to 20 in the production medium GYST supplemented with 100 mg/ml ampicillin to which the IPTG inducer to the final concentration of 0.4 mM was added at the beginning of the fermentation. The fermentation was performed in a batch mode for 20-23 hours at 25°C, 500 rpm and air flow 1 wm (7 L laboratory fermentor). At the end of the process, the OD_{λ600nm} of the culture was approximately 25.

The biomass was centrifuged for 5 minutes at 5000 rpm (Beckman centrifuge) and frozen for further processing. After SDS-PAGE and staining with Coomassie, the proportion of G-CSF in total proteins was determined by using a densitometric analysis. It ranged between 30% and 40%.

### Induction with IPTG at OD₆₀₀ₙₘ ≈6.0

The fermentation was performed in the manner similar to that for the induction with 0.4 mM IPTG at the beginning of the process, except that the IPTG inducer to the final concentration or 0.4 mM was added when the culture reached OD_{λ600nm} m 6.0. The fermentation was performed in a batch mode 18-20 hours at 25°C, 500 rpm and air flow 1 vvm (7 L laboratory fermentor). At the end of the process the OD_{λ600nm} of the culture was approximately 30. The biomass was centrifuged for 5 minutes at 5000 rpm (Beckman centrifuge) and frozen for further processing. After SDS-PAGE and staining with Coomassie, the proportion of G-CSF in total proteins was determined by using the profile densitometric analysis, it ranged between 30% and 40%. The inclusion bodies were isolated from the biomass as described in Example 3. Then, the pellet with the inclusion bodies was washed with cold water or cold 10 mM TrisHCl/pH=8 buffer, centrifuged again for 30 minutes at 10000 rpm and solubilised under non-denaturing conditions with a 50-fold volume of 0.2% N-lauroyl sarcosine in 40 mM TrisHCl/pH=8.0. Solubilisation was left to run overnight for 16 to 18 hours at room temperature under shaking at 100-150 rpm. After solubilisation the concentration of proteins was determined by the Bradford method using pure hMet-G-CSF as a standard.

The concentration of total solubilised proteins ranged between 2 and 4 mg/ml after both induction modes.

### Comparison of the proportion of hG-CSF in the inclusion bodies when IPTG is added at the beginning of the fermentation (instantaneous induction) or when OD₆₀₀ₙₘthe of the culture is approx. 6.0

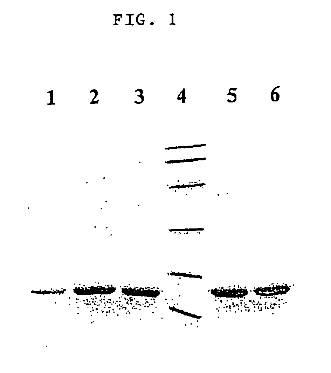

Figure 1 shows the results of the protein composition in the inclusion bodies.
Method: SDS-PAGE (4% stacking gel, 15% separating gel, stained with Coomassie brilliant blue).
The conditions for the preparation of inclusion bodies: cultivation temperature 25°C, performing of fermentation in a batch mode, induction with 0.4 mM IPTG at OD₆₀₀ₙₘ ≈ 6.0 or at the beginning, washing with cold water or cold 10 mM TrisHCl/pH=8.0 buffer, solubilisation in 0.2 % N-lauroyl sarcosine.
Lane 1: hG-CSF standard 0.6 µg
Lane 2: induction at OD₆₀₀ₙₘ ≈ 6.0, inclusion bodies washed with water
Lane 3: induction at OD₆₀₀ₙₘ ≈ 6.0, inclusion bodies washed with 10 mM
   TrisHCl/pH=8.0 buffer.
Lane 4: molecular weight standards (LMW - BioRad)
Lane 5: induction at the beginning, inclusion bodies washed with water
Lane 6: induction at the beginning, inclusion bodies washed with 10 mM
   TrisHCl/pH=8.0 buffer.

The results demonstrate that when IPTG is added to the medium at the beginning, inclusion bodies with very high content of G-CSF and insignificant impurities of other *E. coli* proteins are obtained. In the case of induction at OD₆₀₀ₙₘ ≈ 6.0, the content of G-CSF in the inclusion bodies is still very high but the content of other *E. coli* proteins is higher and may interfere with the subsequent isolation steps.

### Example 2: The influence of temperature on the solubility of inclusion bodies

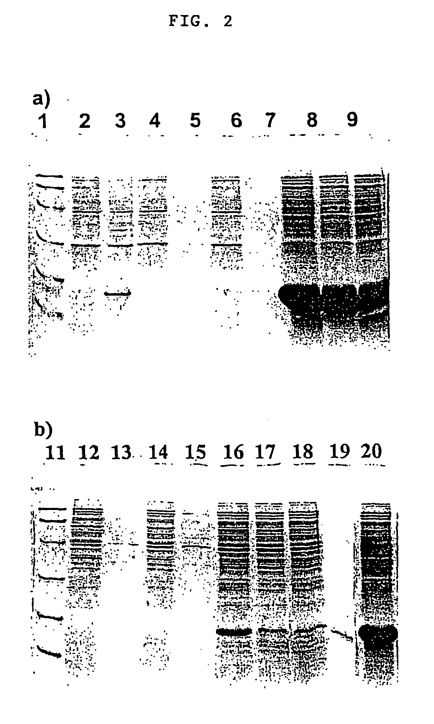

The solubility of inclusion bodies is much increased when the cells are cultivated at 25°C. Washing with detergents would therefore lead to losses of the correctly folded precursor of G-CSF into the washing solution. However, washing should be carried out, at least with water, as otherwise some proteins or other components arising from host cell organism, that may disturb the subsequent purification process such as chromatography, remain adhered to or trapped inside the inclusion bodies and may be subsequently co-solubilised.
Preparation of biomass: *E. coli* culture. BL21(DE3) pET3a/P-Fopt5 from the strain bank at -70°C was inoculated in a ratio 1 to 500 to LBG medium supplemented with 100 mg/ml ampicillin and the culture was cultivated 14-18 hours on a shaker at 150 rpm at 25°C. This culture was used as an inoculation culture to inoculate the fermentor culture in a ratio of 1 to 20 in a production medium GYST supplemented with 100 mg/ml ampicillin to which the IPTG inducer to the final concentration of 0.4 mM was added at the beginning of the fermentation. The fermentation was performed as a batch mode at 500 rpm and air flow 1 vvm (7 L laboratory fermentor) at two different temperatures:
- at 25°C 18-25 hours, at the end of the process the OD_{λ600nm} of the culture was approx. 25.
- at 37°C 8-25 hours, at the end of the process the OD_{λ600nm} of the culture was approx. 28.
Isolation of non-classical inclusion bodies: After the completed fermentation, the bacterial pellet was separated from the supernatant by centrifugation at +4°C and 5000 rpm. The wet bacterial pellet was resuspended in a 4-fold volume of buffer X (10 mM Tris/HCl, pH=8,0). The homogenisation of the sample was performed by using Ultraturax. The cells were then broken by using the homogeniser EmulsiFlex C-5 (AVESTIN) in one passage at the pressure difference of 10000 to 15000 psi (70 -110 MPa). After the 30-minute centrifugation at 10000 rpm the supernatant comprising the soluble bacterial *E. coli* proteins was discarded, and the pellet (comprising non-classical inclusion bodies) was frozen at -20°C and used for non-classical inclusion bodies washing and solubilisation experiments.
The following experiments of non-classical inclusion bodies washing were performed (Figure 2):
A. Washing of inclusion bodies with water
   The determined quantity of non-classical inclusion bodies was resuspended in a 10-fold volume of cold water (+4°C), centrifuged for 10 minutes at 10000 rpm at +4°C and the process was repeated with the same volume of cold water. In the supernatants after both washings the amount of proteins was determined according to the Bradford method (using bovine serum albumin (BSA) as a standard). The protein composition was analysed by SDS-PAGE.
   Water quality: The water was prepared by using Milli-Q RG (Millipore) apparatus.
B. Washing of inclusion bodies with buffer X (10mM Tris/HCl, pH 8.0)
   The determined quantity of non-classical inclusion bodies was resuspended in a 10-fold volume of cold buffer X (10 mM Tris/HCl, pH 8.0) (+4°C), centrifuged for 10 minutes at 10000 rpm at +4°C and the process was repeated with the same volume of cold buffer X. In the supernatants after both washings the amount of proteins was determined according to the Bradford method (using BSA as a standard). The protein composition was analysed by SDS-PAGE.
C. Washing of inclusion bodies with 1% Na-deoxycholate
   The determined quantity of non-classical inclusion bodies was resuspended in a 10-fold volume of cold (+4°C) buffer W (50 mM Tris/HCl, pH 9.0 with 1% Na deoxychyolate, 5 mM dithiothreitol (DTT and 5 mM EDTA), left for 30 minutes on ice and centrifuged for 10 minutes at 10000 rpm at +4°C. In the supernatants the amount of proteins was determined according to the Bradford method (using BSA as a standard). The protein composition was analysed by SDS-PAGE.
D. Washing of inclusion bodies with 1%Triton X-100
   The determined quantity of non-classical inclusion bodies was resuspended in a 10-fold volume of cold (+4°C) buffer X (10 mM Tris/HCl, pH=8.0) with 1% Triton X-100, left for 30 minutes on ice and centrifuged for 10 minutes at 10000 rpm at +4°C. In the supernatants the amount of proteins was determined according to the Bradford method (using BSA as a standard). The protein composition was analysed by SDS-PAGE.
E. Washing of inclusion bodies with 2 M urea
   The determined quantity of non-classical inclusion bodies was resuspended in a 10-fold volume of cold (+4°C) buffer X (10 mM Tris/HCl, pH=8.0) with 2 M urea, left for 30 minutes on ice and centrifuged for 10 minutes at 10000 rpm at +4°C. In the supernatants the amount of proteins was determined according to the Bradford method (using BSA as a standard). The protein composition was analysed by SDS-PAGE.
F. Washing of inclusion bodies with 8 M urea
   The determined quantity of non-classical inclusion bodies was resuspended in a 10-fold volume of cold (+4°C) buffer X (10 mM Tris/HCl, pH=8.0) with 8 M urea, left for 30 minutes on ice and centrifuged for 10 minutes at 10000 rpm at +4°C. In the supernatants the amount of proteins was determined according to the Bradford method (using BSA as a standard).

The protein composition was analysed by SDS-PAGE. Figure 2 shows the comparison of the solubilisation of the inclusion bodies regarding the cultivation temperature (25°C or 37°C).
Method: SDS-PAGE (4% stacking gel, 15% separating gel, stained with Coomassie brilliant blue).
The conditions for the preparation of non-classical inclusion bodies: cultivation temperature: 25°C (lanes 4-10) and 37°C (lanes 12-18, 20), performing of fermentation in a batch mode, induction with 0.4 mM IPTG at the beginning, washing with cold water or cold 10 mM TrisHCl/pH=8.0 buffer or 1% Na-deoxycholate or 1% Triton X-1 00 or 2 M urea or 8 M urea.
Lane 1: molecular weight standards (LMW - BioRad)
Lane 2: the soluble fraction of proteins (homogenate supernatant)
Lane 3: total proteins
Lane 4: solubilised proteins after the first washing of the non-classical inclusion bodies with water
Lane 5: solubilised proteins after the second washing of the non-classical inclusion bodies with water.
Lane 6: solubilised proteins after the first washing of the non-classical inclusion bodies with 10 mM TrisHCl/pH=8.0
Lane 7: solubilised proteins after the second washing of the non-classical inclusion bodies with 10 mM TrisHCl/pH=8.0
Lane 8: solubilised proteins after washing of the non-classical inclusion bodies with 1% Na-deoxycholate
Lane 9: solubilised proteins after washing of non-classical inclusion bodies with 2 M urea
Lane 10: solubilised proteins after washing of nort-classical inclusion bodies with 1% Triton-X100
Lane 11: molecular weight standards (LMW - BioRad)
Lane12: solubilised proteins after the first washing of the non-classical inclusion bodies with water
Lane 13: solubilised proteins after the second washing of non-classical inclusion bodies with water
Lane 14: solubilised proteins after the first washing of non-classical inclusion bodies with 10 mM TrisHClpH=8.0
Lane 15: solubilised proteins after the second washing of non-classical inclusion bodies with 10 mM TrisHCl/pH=8.0
Lane 16: solubilised proteins after washing of non-classical inclusion bodies with 1% Na-deoxycholate
Lane 17: solubilised proteins after washing of non-classical inclusion bodies with 2 M urea
Lane 18: solubilised proteins after washing of non-classical inclusion bodies with 1% Triton-X100
Lane 19: standard hG-CSF, 0.6 µg
Lane 20: solubilised proteins after washing of non-classical inclusion bodies with 8 M urea

As shown in Figure 2, the solubility of the non-classical inclusion bodies was more increased when the cells were cultivated at 25°C (lanes 4-10) than when the cells were cultivated at 37°C (lanes: 12-18, 20). When the cells were cultivated at 25°C, washing with the detergents (1% deoxycholate or 1% Triton-X100) and with 2 M urea was no longer possible as the majority of heterologous protein was lost with the washing solution (lanes 8, 9, 10), in the case the cells were cultivated at 37°C washing with the detergents (1% deoxycholate, lane 16), 2 M urea (lane 17) and 1% Triton-X100 (lane 18) was still possible. As seen, the non-classical inclusion bodies obtained at the cultivation temperature 37°C could be solubilised only with 8 M urea (lane 20).

### Example 4: Influence of the performing of fermentation on accumulation of G-CSF and on proportion of correctly folded precursor of G-CSF in inclusion bodies

High proportion of correctly folded precursor of G-CSF in non-classical inclusion bodies can be obtained by performing the fermention in a batch mode or fed-batch mode.

### Performing fermentation in a batch mode

*E. coli* BL21 (DE3) pET3a/P-Fopt5 from the strain bank at -70°C was inoculated in a ratio 1 to 500 to LBG medium supplemented with 100 mg/ml ampicillin and the culture was cultivated 14-18 hours on a shaker at 25°C, 150 rpm. This culture was used as an inoculation culture to inoculate the fermentor culture in a ratio 1 to 20 in the production medium GYST supplemented with 100 mg/ml ampicillin to which the IPTG inducer to the final concentration of 0.4 mM was added at the beginning of the fermentation or in the early exponential phase when OD_{λ600nm} of the culture was 6. The fermentation was performed in a batch mode 18-25 hours at 25°C, 500 rpm and air flow 1 wm (7 L laboratory fermentor). At the end of the process the OD_{λ600nm} of the culture was approximately 25 at the beginning of induction and 30 in the case of later induction. The biomass was centrifuged for 5 minutes at 5000 rpm (Beckman centrifuge) and frozen for the further processing. After SDS-PAGE and staining with Coomassie, the proportion of G-CSF in total proteins was determined by using a densitometric analysis. It.ranged between 30% and 40%.

### Performing of fermentation in a fed-batch mode

The fermentation was performed in a fed-batch mode 25.5-30 hours at 25_{°}C, 500 rpm and air flow 1 wm (7 L laboratory fermentor). Once the glucose in the medium was consumed, that is, at the end of the batch process (performed as described above), and the pH of the medium increased, feeding a 20% glucose solution with 100 mg/l ampicillin was initiated to provide the specific growth rate µ between 0.05 and 0.1 hr⁻¹. The process was completed after 7-7.5 hours of feeding (totally 25.5-30 hours) when the OD_{λ600nm} of the culture was approximately 42. The biomass was centrifuged for 5 minutes at 5000 rpm (Beckman centrifuge) and frozen for further processing. After SDS-PAGE and staining with Coomassie, accumulation of G-CSF, maintained at a similar high level as in the case of performing the fermentation in a batch mode, was determined by using a densitometric analysis. It ranged between 30% and 40%. The non-classical inclusion bodies were isolated from the biomass, as described in Example 3. Then, the pellet with non-classical inclusion bodies was washed with cold water, centrifuged again for 30 minutes at 10000 rpm and solubilised under non-denaturing conditions in a 50-fold the volume of 0.2 % N-lauroyl sarcosine in 40 mM TrisHCl/pH=8.0. Solubilisation was left to run overnight for 16 to 18 hours at room temperature under shaking at 100-150 rpm. After solubilisation the concentration of proteins was determined by the Bradford method using pure hMet-G-CSF as a standard.

Concentrations of the proteins ranged between 2 and 4 mg/ml after both principles of performing the fermentation. The proportion of correctly folded precursor of G-CSF or biologically active G-CSF was determined by measuring biologically activity of the solubilised non-classical inclusion bodies without removal of N-lauroyl sarcosine (at this dilution N-lauroyl sarcosine does not interfere with the biological activity measurement).

Table 2 below shows the proportion of correctly folded precursor of G-CSF depending on the way of performing the fermentation and the induction mode

**Table 2**

| Principle of performing the fermentation | Induction with 0.4 mM IPTG | Proportion of biologically active G-CSF |
|---|---|---|
| batch | at the beginning | 44% |
| fed-batch | at the beginning | 39% |
| batch | OD₆₀₀ₙₘ ≈ 6 | 43% |
| fed-batch | OD₆₀₀ₙₘ ≈ 6 | 37% |

As evident from the data presented in Table 2, the high proportion of the biologically active G-CSF was obtained in all cases, being the highest by performing the fermentation in a batch mode.

### Example 5: Influence of the induction with lactose on accumulation of G-CSF and proportion of correctly folded precursor of G-CSF

*E. coli* BL21 (DE3) pET3a/P-Fopt5 from the strain bank at -70°C was inoculated in a ratio 1 to 500 to LBG medium supplemented with 100 mg/ml ampicillin and the culture was cultivated 14-18 hours on a shaker at 25°C, 150 rpm. This culture was used as an inoculation culture to inoculate the fermentor culture in a ratio 1 to 20 in the modified production medium LYST supplemented with 100 mg/ml ampicillin wherein glycerol (6 g/l) and lactose (2 g/l or 4 g/l) were used instead of glucose as the source of carbon which simultaneously were also the expression inducer instead of IPTG. The fermentation was performed as a batch mode for 17-21 hours at 25°C, 500 rpm and air flow 1 wm (7 L laboratory fermentor). At the end of the process the OD_{λ600nm} of the culture was approximately 20. The biomass was centrifuged for 5 minutes at 5000 rpm (Beckman centrifuge), washed 1x with 10 mM Tris/HCl, pH=8 buffer and frozen for the further processing. After SDS-PAGE and staining with Coomassie, accumulation of G-CSF was determined by using a densitometric analysis, which was 27% for induction with 2 g/l lactose and 33% for induction with 4g/l lactose. The non-classical inclusion bodies were isolated from the biomass, as described in Example 3. The pellet with non-classical inclusion bodies was washed with cold water, centrifuged again for 30 minutes at 10000 rpm and solubilised under non-denaturing conditions in a 50-fold the volume of 0.2 % N-lauroyl sarcosine in 40 mM TrisHCl/pH=8.0. Solubilisation was left overnight for 16 to 18 hours at room temperature under shaking at 100-150 rpm.

Table 3 shows the proportion of correctly folded precursor of G-CSF or proportion of biologically active G-CSF (in %) in the non-classical inclusion bodies obtained by induction of the production strain *E. coli* BL21 (DE3) pET3a/P-Fopt5 with lactose.

**Table 3**

| Expression system | Induction with lactose | Cultivation temperature | Proportion of biologically active G-CSF |
|---|---|---|---|
| pET3a / P-Fopt5 *E. coli* BL21 (DE3) | 2 g/l | 25°C | ≈ 25% |
| pET3a / P-Fopt5 *E. coli* BL21 (DE3) | 4 g/l | 25°C | ≈ 23% |

As evident from Table 3, using the induction with lactose, at least a 23% proportion of the correctly folded precursor of G-CSF in inclusion bodies was obtained when cultivated at 25°C.

### Example 6: Influence of the induction with NaCl on accumulation of G-CSF and on proportion of correctly folded precursor of G-CSF

*E. coli* culture BL21 (DE3) pET3a/P-Fopt5 from the strain bank at -70°C was inoculated in a ratio 1 to 20 in the LBON medium supplemented with 100 mg/ml ampicillin and cultivated over the day for 8 hours on a shaker at 25°C, 160 rpm. 1 ml of this culture was used as an inoculation culture to inoculate 20 ml of GYSPON medium supplemented with 100 mg/ml ampicillin to which the NaCl inducer to the final concentration of 1.2 M was added at the beginning or at the OD_{λ600nm} ≈ 0.5, that is, after approximately 3 hours of cultivation. In both cases the culture was cultivated on a shaker at 25°C and 160 rpm for 20-24 hours. For SDS-PAGE analysis, after completed cultivation 5 ml of this culture was centrifuged for 5 minutes at 5000 rpm. The pellets were then resuspended in 15 ml of 10 mM TrisHCl/pH=8.0. The samples were mixed in a ratio 3 to 1 with 4x SDS - sample buffer with DTT (pH=8.7) and heated at 95°C for 10 minutes, centrifuged and the supernatant was loaded to the gel. The accumulation of G-CSF is presented in Table 4 which shows the comparison of accumulation of G-CSF of the production strain BL21-SI pET3a/P-Fopt5 in different media and induction modes.. As shown in the table, higher accumulation of G-CSF was obtained when 1.2 M NaCl was added to the medium at the beginning, that is, at inoculation and not conventionally at OD_{λ600nm} ≈ 0.5, as recommended by the producer (Life Technologies).

**Table 4**

| Expression system | Cultivation and induction conditions | Cultivation temperature | Accumulation of G-CSF |
|---|---|---|---|
| pET3a / P-Fopt5 *E. coli* BL21-SI | GYSPON supplemented with 100 mg/ml ampicillin 1.2 M NaCl at the beginning | 25°C | 25% |
| pET3a / P-Fopt5 *E. coli* BL21-SI | GYSPON supplemented with 100 mg/ml ampicillin 1.2 M NaCl at OD_{λ600nm} ≈ 0.5, | 25°C | 17% |

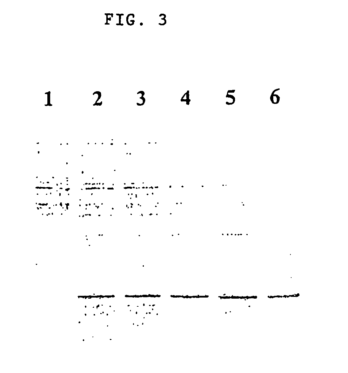

The values reported for the G-CSF assay were obtained by the densitometric analysis of SDS-PAGE gels stained with Coomassie brilliant blue shown in Figure 3. Relative proportion was determined by the profile analysis (program Molecular analyst; BioRad) of gels on the apparatus Imaging densitometer Model GS670 (BioRad).
Figure 3 shows the composition of proteins in the non-classical inclusion bodies. Method: SDS-PAGE (4% stacking gel, 15% separating gel, stained with Coomassie brilliant blue).
The conditions for the preparation of non-classical inclusion bodies: production strain BL21-SI pET3a/P-Fopt5, cultivation temperature 25°C, fermentation in shake flasks, non-induced culture or induction with 1.2 M NaCl at OD₆₀₀ₙₘ ≈ 0.5 or with the induction at the beginning.
Lane 1: BL21 (SI) pET3a/P-Fopt5, non-induced culture
Lane 2: BL21 (SI) pET3a/P-Fopt5, induction with 1.2 M NaCl at OD₆₀₀ₙₘ ≈ 0.5
Lane 3: BL21 (SI) pET3a/P-Fopt5, induction with 1.2 M NaCl at OD₆₀₀ₙₘ ≈ 0.5
Lane 4: BL21 (SI) pET3a/P-Fopt5, induction with 1.2 M NaCl at the beginning
Lane 5: BL21 (SI) pET3a/P-Fopt5, induction with 1.2 M NaCl at the beginning
Lane 6: rhG-CSF 0.6 µg

### Preparation of the samples for determination of biological activity and non-classical inclusion bodies washing experiments

For the analysis of biological activity and non-classical inclusion bodies washing experiments, a larger quantity of biomass was prepared. E. coli culture BL21-SI pET3a/P-Fopt5 from the strain bank at -70°C was inoculated in a ratio 1 to 20 in the LBON medium supplemented with 100 mg/ml ampicillin and cultivated over the day for 8 hours on a shaker at 25°C, 160 rpm. 10- ml aliquots of this culture were incubated in 8 x 200 ml of GYSPON medium supplemented with 100 mg/mi ampicillin to which the NaCl inducer to the final concentration of 1.2 M was added already at the beginning. The culture was cultivated on a shaker at 25°C and 160 rpm for 24 hours. The non-classical inclusion bodies were isolated from the biomass, as described in Example 3. Then, the pellet with non-classical inclusion bodies was washed with cold water and solubilised with 0.2 % N-lauroyl sarcosine as described in Example 9. After solubilisation, the protein concentration was determined according to the Bradford method using pure hMet-G-CSF as a standard. The concentration of total solubilised proteins ranged between 2 and 3 mg/ml.

Thus, Table 5 below shows the proportion of correctly folded precursor of G-CSF or biologically active G-CSF in non-classical inclusion bodies obtained by the induction of a production strain *E*. *coli* BL21-SI pET3a / P-Fopt5 with 1.2 M NaCl.

**Table 5**

| Expression system | Cultivation temperature | Proportion of biologically active G-CSF |
|---|---|---|
| pET3a / P-Fopt5 *E. coli* BL21-SI | 25°C | ≈ 40% |

As evident from Table 5, a high proportion of correctly folded precursor of G-CSF (or biologically active G-CSF) was obtained with a production strain *E. coli* BL21-SI pET3a/P-Fopt5 by induction of the expression of heterologous gene with 1.2 M NaCl when cultivated at 25°C.

### Solubility of non-classical inclusion bodies

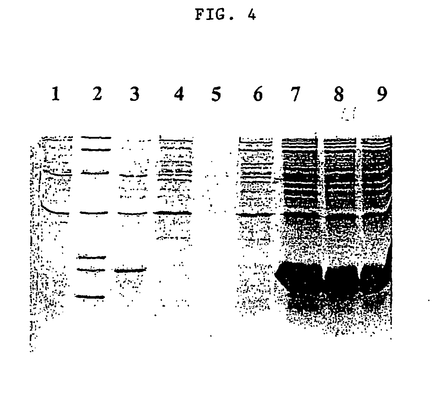

The solubility of inclusion bodies was much increased with a production strain *E. coli* BL21-SI pET3a/P-Fopt5 by induction of the expression of the heterologous gene with 1.2 M NaCl when cultivated at 25°C. Here, too, washing of the non-classical inclusion bodies with detergents would lead to losses of the correctly folded precursor of G-CSF in the washing solution. This is demonstrated by Figure 4 which shows the comparison of the solubility of the non-classical inclusion bodies with regard to washing with different solvents.
Method: SDS-PAGE (4% stacking gel, 15% separating gel, stained with Coomassie brilliant blue).
The conditions for the preparation of non-classical inclusion bodies: strain BL21 (SI) pET3a/P-Fopt5, cultivation temperature 25°C, fermentation in shake flasks, induction with 1.2 M NaCl, washing with water or 10 mM TrisHCI/pH=8.0 or 1 % Na-deoxycholate or 1 % Triton X-100 or 2 M urea.
Non-classical inclusion bodies were prepared in shake flasks at 25°C, induced with 1.2 mM NaCl added in the medium at the beginning.
Lane 1: soluble fraction of the proteins (homogenate supernatant)
Lane 2: molecular weight standards (LMW - BioRad)
Lane 3: total proteins
Lane 4: solubilised proteins after the first washing of non-classical inclusion bodies with water
Lane 5: solubilised proteins after the second washing of non-classical inclusion bodies with water
Lane 6: solubilised proteins after the first washing of the non-classical inclusion bodies with 10 mM TrisHCl/pH=8.0
Lane 7: solubilised proteins after washing of non-classical inclusion bodies with 1% Na-dexycholate
Lane 8: solubilised proteins after washing of non-classical inclusion bodies with 2 M urea
Lane 9: solubilised proteins after washing of non-classical inclusion bodies with 1% Triton-X100

### Example 7: Influence of the additives capable of causing stress, e.g., ethanol or Propanol, on proportion of correctly folded precursor of G-CSF in inclusion bodies

*E. coli* BL21 (DE3) pET3a/P-Fopt5 from the strain bank at -70°C was inoculated in a ratio 1 to 500 in the LBPGmedium (10 g/l phytone, 5 g/l yeast extract, 10 g/l NaCl), supplemented- with 100 mg/ml ampicillin and cultivated for 17 hours on a shaker at 25°C, 160 rpm. 10 ml of this culture was inoculated in 200 ml of the medium with instantaneous addition of the IPTG inducer to the final concentration of 0.4 mM:
- GYSP medium supplemented with 100 mg/ml ampicillin (control);
- GYSP medium supplemented with 100 mg/ml ampicillin with added ethanol (final ethanol concentration in the medium 3%);
- GYSP medium supplemented with 100 mg/ml ampicillin with added iso-propanol (final iso-propanol concentration in the medium 3%).

The culture was then cultivated on a shaker at 25°C and 160 rpm for 24 hours in the case of the control (GYSP medium supplemented with 100 mg/ml ampicillin). In the case of GYSP medium supplemented with 100 mg/ml ampicillin with added ethanol or iso-propanol, the cultures were cultivated under the same conditions for 34 hours, as addition of the either additive slowed the growth. Non-classical inclusion bodies were isolated from the biomass, as described in Example 3. Then, the pellet containing the non-classical inclusion bodies was washed with cold water and solubilised with addition of 0.2% N-lauroyl sarcosine as described in Example 9.

Thus, Table 6 shows the proportion of correctly folded precursor of G-CSF (in %) in non-classical inclusion bodies obtained by the induction of a production strain *E. coli* BL21 (DE3) pET3a / P-Fopt5 in GYSP medium supplemented with 100 mg/ml ampicillin with 0.4 mM IPTG and with addition of ethanol or propanol which are capable of causing stress.

**Table 6**

| Expression system | Cultivation temperature in °C | Additive | Proportion of biologically active G-CSF |
|---|---|---|---|
| pET3a / P-Fopt5 *E. coli* BL21 (DE3) | 25°C | Without additive | ≈ 50% |
| pET3a / P-Fopt5 *E. coli* BL21 (DE3) | 25°C | 3% ethanol | ≈ 59% |
| pET3a/P-Fopt5 *E. coli* BL21 (DE3) | 25°C | 3 % propanol | ≈ 62% |

As evident from Table 6, the additions of ethanol or propanol, which trigger stress proteins, increase the proportion of correctly folded precursor of G-CSF or biologically active G-CSF in non-classical inclusion bodies.

### Example 8: Solubilisation of non-classical inclusion bodies with urea in buffer

12 ml of buffer Z (40 mM Tris/HCl pH=8.0) with 2 M urea was added to 0.30 g of wet non-classical inclusion bodies which were previously washed with cold water (see Example 3). After homogenisation the non-classical inclusion bodies were solubilised for 16 hours at 20°C under gentle shaking at 80 rpm. The solution was centrifuged for 10 minutes at 14000 rpm and +10°C and the pellet was discarded The supernatant containing solubilised non-classical inclusion bodies was used for determination of the total protein content, SDS-PAGE analyses and for determination of biological activity.

The protein concentration according to the Bradford method (using hMetG-CSF as a standard): 2.6 mg/ml, total solubilised proteins: 31 mg, (the amount of solubilised proteins: approximately 10% in regard to the mass of wet non-classical inclusion bodies).

Biological activity of G-CSF: about 1.5 x 10⁷ IU/mg

### Example 9: Solubilization of non-classical inclusion bodies with N-laurovl-sarcosine in buffer

15.6 ml of buffer Z (40 mM Tris/HCl pH=8.0) with 0.2% N-lauroyl-sarcosine was added to 0.31 g of wet non-classical inclusion bodies which were previously washed with cold water (see Example 3). After homogenisation the non-classical inclusion bodies were solubilised for 1 hour at room temperature (20-22°C) under shaking at 100-50 rpm. 0.1 M CuSO₄ 5H₂O to the final concentration of 40 µM was added to promote oxidation (i.e. disulphide bonds formation). Shaking was left overnight (16 hours) at 80 rpm and at 20°C. Next day the solution was centrifuged for 10 minutes at 14000 rpm and +10°C and the pellet was discarded. N-lauroyl-sarcosine was removed from the supernatant containing the solubilised non-classical inclusion bodies by using DOWEX (DOWEX 1x4 Sigma), in such a way that 0.39 g DOWEX was added, the solution was shaken for 1 hour at room temperature (20-22°C) and 100-150 rpm. The pellet was discarded. The solution of solubilised non-classical inclusion bodies was used for determination of total protein content, SDS-PAGE analysis and for determination of biological activity. Protein concentration according to the Bradford method (using hMetG-CSF as a standard): 4.4 mg/ml, total solubilised protein content: 68 mg, (the amount of solubilised proteins; approx. 20% in regard to the mass of wet non-classical inclusion bodies.

Biological activity of G-CSF: about 4.0 - 4.5 x10⁷ IU/mg - independent of adding CuSO₄x 5H₂O.

### Example 10: Solubilisation of non-classical inclusion bodies with NDSB in buffer

A 40-fold surplus of buffer Z (40 mM Tris/HCl pH=8.0) with addition of different NDSB (non-detergent sulfobetaine) at the concentration of 0.2% was added to 0.16-g aliquots of wet non-classical inclusion bodies which were previously washed with cold water (see Example 3). NDSB 195, NDSB 201, NDSB 211 and NDSB 256 were used. After homogenisation, the samples of non-classical inclusion bodies were solubilised at 20°C under shaking at 80 rpm overnight. In parallel experiments, after an initial 30-minute solubilisation period, 0.1 M CuSO₄ x 5H₂O was added to a final concentration of 40 µM to promote oxidation. Next day after centrifugation, the undissolved pellets were discarded, and the supernatants, without any prior processing, were used for determination of total protein content, SDS-PAGE analysis and for determination of biological activity. The protein concentration according to the Bradford method (using hMetG-CSF as a standard): about 3.6 mg/ml in all cases, thus, the total protein content: about 24 mg, (the amount of solubilised proteins: approximately 15% in regard to the mass of wet non-classical inclusion bodies).

Biological activity of G-CSF: about 3 - 4 x10⁷ IU/mg - independent of adding CuSO₄ 5H₂O.

### Example 11: Solubilisation of non-classical inclusion bodies with DMSO in buffer

A 40-fold surplus of buffer Z (40 mM Tris/HCl pH=8.0) with 5% BMSO was added to 0.16 g of non-classical inclusion bodies which were previously washed with cold water (see Example 3). After homogenisation, non-classical inclusion bodies were solubilised at 20°C under shaking at 80 rpm overnight. In a parallel experiment, after an initial 30-minute solubilisation period, 0.1 M CuSO₄ x 5H₂O was added to a final concentration of 40 µM to promote oxidation. Next day after centrifugation the precipitates werediscarded , and the supernatants, without any prior processing, were used for determination of total protein content, SDS-PAGE analysis and for determination of biological activity. The protein concentration according to the Bradford method (using hMetG-CSF as a standard): about 3.6 mg/ml, thus, the total protein content: about 24 mg, (the amount of solubilised proteins: approximately 15% in regard to mass of wet non-classical inclusion bodies).

Biological activity of G-CSF: about 4 x10⁷ IU/mg - independent of adding CuSO₄ x 5H₂O.

### Example 12: Testing of biological activity of G-CSF in vitro

The biological activity of G-CSF was determined by using the proliferation assay on the cell line NFS-60 according to the known method (Hammerling U arid co-workers in J Pharm Biomed Anal 13, 9-20 (1995)) and use of the international standard Human recombinant G-CSF (881502, yeast cell derived; NIBSC Potters Bar, Hertfordshire, UK); (Mire-Sluis A R and co-workers in J Immunol Methods 179, 117-126 (1995)).

## Claims

1. A process for the production of G-CSF which involves the expression of said G-CSF as a heterologous protein in E. coli, wherein the correctly folded precursor of the G-CSF-after expression is accumulated in inclusion bodies, wherein performing the biosynthesis comprises adjusting the temperature of cultivation to between 20°C and 30°C, and wherein performing the biosynthesis comprises adjustment of the induction mode comprising selecting the inducer from the group consisting of (a) IPTG in a concentration in the range from 01. mM to 1 mM, (b) lactose in a concentration in the range of between 1 and 10 g/l, and (c) NaCl in the range from 0.3 M to 1.3 M.

2. The process according to claim 1, wherein the process involves the way of performing the biosynthesis of G-CSF comprising adjusting one or more parameters which are selected from the group consisting of composition of the cultivation medium, induction mode, principle of performing the fermentation, addition of an agent capable of causing stress, and co-expression of auxiliary proteins.

3. The process according to any of claims 1 or 2, wherein the temperature of cultivation is about 25 °C..

4. The process according to any of claims 1 to 3, wherein the selected inducer is IPTG.

5. The process according to claim 4, wherein the concentration of IPTG is 0.3 mM to 0.6 mM.

6. The process according to claim 4, wherein the concentration of IPTG is about 0.4 mM.

7. The process according to claim 4, wherein the concentration of IPTG is about 1 mM.

8. The process according to any of claims 1 to 3, wherein the selected inducer is lactose and wherein the concentration of lactose is in the range between 2 and 4 g/l.

9. The process according to claim8, wherein the selected inducer is NaCl and wherein the concentration of NaCl is 1.0 to 1.3 M

10. The process according to any one of claims 1 to 9, wherein the adjustment of the induction mode comprises adding the inducer at the beginning of the fermentation.

11. The process according to any one of claims 1 to 10, wherein the inducer is added instantaneously subsequent to the preculturing step, if a preculturing step is performed.

12. The process according to any one of claims 1 to 11, wherein the principle of performing the biosynthesis is selected from the group comprising: performing of fermentation in a batch mode, performing of fermentation in a fed batch mode and fermentation in shake flasks.

13. The process according to claim 12, wherein the selected principle of performing the fermentation is a batch mode.

14. The process according to any one of claims 1 to 13, wherein the medium is selected from the group comprising: GYST, GYSP, LYSP, LYST, LBON and GYSPON.

15. The process according to claim 14 wherein the selected medium is GYST or GYSP.

16. The process according to any one of claims 2 to 15, wherein the additive which is capable of causing stress is selected from the group consisting of ethanol and propanol.

17. The process according to any one of claims 1 to 16, further comprising washing of the inclusion bodies.

18. The process according to claim 17, wherein the washing is performed by using a solution which is selected from the group consisting of Tris/HCl buffer, phosphate buffer, acetate buffer, citrate buffer and water.

19. The process according to claim 18, wherein the concentration of the selected buffer is in the range from 1 mM to 10 mM.

20. The process according to claim 18, wherein the selected solution is water.

21. The process for production of a protein according to any one of claims 1 to 20, wherein the process further comprises solubilisation of the inclusion bodies.

22. The process according to claim 21, wherein the solubilisation is performed by using an agent for solubilisation being selected from the group consisting of: urea in non-denaturating concentrations (1-2 M), N-lauryl sarcosine in non-denaturating concentrations (0.05-0. 25% (m/v)), Zwittergents in low, non-denaturating concentrations, non-detergent sulfobetains (NDSBs), betain, sarcosine, carbamoyl sarcosine, taurine, DMSO and a buffer in a high, solubilising concentration, wherein the buffer is selected from the group consisting of: HEPES, HEPPS, MES, ACES, and MES.

23. The process according to claim 22, wherein the selected solvent is N-lauroyl-sarcosine.

24. The process according to claim 23, wherein the concentration of N-lauryl sarcosine is in the range from 0.1 % to 0.25%.

25. The process according to claim 24, wherein the concentration of N-lauryl sarcosine is about 0.2%.

26. The process for production of biologically active G-CSF by biosynthesis, in E. coli wherein parameters for the way of performing the biosynthesis are selected as follows:
• cultivation temperature: 20-30°C, preferably about 25°C .
• composition of cultivation medium: selected from group consisting of GYST, GYSP, LYSP, LYST, LBON and GYSPON, preferably GYST and GYSP .
• type of fermentation: batch mode
• induction mode: adding IPTG, in case of an optional preculturing step after said preculturing step, at the beginning of the fermentation, to adjust a final IPTG concentration in the range of 0.3 to 0.6 mM, preferably to about 0.4 mM.

27. The process for production of biologically active G-CSF according to claim 26, wherein the process further comprises isolating inclusion bodies and washing the isolated inclusion bodies with water.

28. The process for production of biologically active G-CSF according to claim 27 or 28, wherein the process further comprises the solubilisation of inclusion bodies with N-lauryl sarcosine with the concentration of 0.1 to 0.25%, preferably about 0.2%.

29. The process according to any of claims 1 to 28, comprising the additional step of using the G-CSF for the preparation of a medicament

## Patentansprüche

1. Verfahren zur Herstellung von G-CSF, das die Expression des G-CSF als heterologes Protein in E. coli umfasst, wobei der korrekt gefaltete Vorläufer des G-CSF nach Expression in Einschlusskörpern akkumuliert wird, wobei das Durchführen der Biosynthese das Einstellen der Temperatur der Kultivierung auf zwischen 20 und 30 °C umfasst und wobei das Durchführen der Biosynthese die Einstellung des Induktionsmodus umfasst, der ein Auswählen des Inducers aus der Gruppe umfasst, die aus (a) IPTG in einer Konzentration im Bereich von 0,1 mM bis 1 mM, (b) Lactose in einer Konzentration im Bereich von zwischen 1 und 10 g/l und (c) NaCl in einem Bereich von 0,3 M bis 1,3 M besteht.

2. Verfahren nach Anspruch 1, worin das Verfahren die Art der Durchführung der Biosynthese von G-CSF umfasst, die ein Einstellen eines oder mehrerer Parameter umfasst, die aus der Gruppe ausgewählt sind, die aus Zusammensetzung des Kultivierungsmediums, Induktionsmodus, Prinzip der Durchführung der Fermentation, Zugabe eines Mittels mit der Fähigkeit zur Hervorrufung von Stress und Co-Expression von Hilfsproteinen besteht.

3. Verfahren nach einem der Ansprüche 1 oder 2, worin die Temperatur der Kultivierung etwa 25 °C beträgt.

4. Verfahren nach einem der Ansprüche 1 bis 3, wobei der ausgewählte Inducer IPTG ist.

5. Verfahren nach Anspruch 4, wobei die Konzentration von IPTG 0,3 mM bis 0,6 mM beträgt.

6. Verfahren nach Anspruch 4, wobei die Konzentration von IPTG etwa 0,4 mM beträgt.

7. Verfahren nach Anspruch 4, wobei die Konzentration von IPTG etwa 1 mM beträgt.

8. Verfahren nach einem der Ansprüche 1 bis 3, wobei der ausgewählte Inducer Lactose ist und wobei die Konzentration von Lactose in einem Bereich von zwischen 2 und 4 g/l liegt.

9. Verfahren nach Anspruch 8, wobei der ausgewählte Inducer NaCl ist und wobei die Konzentration von NaCl 1,0 bis 1,3 M beträgt.

10. Verfahren nach einem der Ansprüche 1 bis 9, wobei die Einstellung des Induktionsmodus die Zugabe des Inducers zu Beginn der Fermentation umfasst.

11. Verfahren nach einem der Ansprüche 1 bis 10, wobei der Inducer augenblicklich nach der Vorkultivierungsstufe, wenn eine Vorkultivierungsstufe durchgeführt wird, zugegeben wird.

12. Verfahren nach einem der Ansprüche 1 bis 11, wobei das Prinzip der Durchführung der Biosynthese aus der Gruppe ausgewählt ist, die ein Durchführen einer Fermentation in einem Batch-Modus, ein Durchführen einer Fermentation in einem Fed-Batch-Modus und eine Fermentation in Schüttelkolben umfasst.

13. Verfahren nach Anspruch 12, wobei das ausgewählte Prinzip der Durchführung der Fermentation ein Batch-Modus ist.

14. Verfahren nach einem der Ansprüche 1 bis 13, wobei das Medium aus der Gruppe ausgewählt ist, die GYST, GYSP, LYSP, LYST, LBON und GYSPON umfasst.

15. Verfahren nach Anspruch 14, wobei das ausgewählte Medium GYST oder GYSP ist.

16. Verfahren nach einem der Ansprüche 2 bis 15, wobei das Additiv, das in der Lage ist, Stress hervorzurufen, aus der Gruppe ausgewählt ist, die aus Ethanol und Propanol besteht.

17. Verfahren nach einem der Ansprüche 1 bis 16, das ferner ein Waschen der Einschlusskörper umfasst.

18. Verfahren nach Anspruch 17, wobei das Waschen durch Verwendung einer Lösung durchgeführt wird, die aus der Gruppe ausgewählt ist, die aus Tris/HCl-Puffer, Phosphatpuffer, Acetatpuffer, Citratpuffer und Wasser besteht.

19. Verfahren nach Anspruch 18, wobei die Konzentration des ausgewählten Puffers in einem Bereich von 1 mM bis 10 mM liegt.

20. Verfahren nach Anspruch 18, wobei die ausgewählte Lösung Wasser ist.

21. Verfahren zur Herstellung eines Proteins nach einem der Ansprüche 1 bis 20, wobei das Verfahren ferner ein Solubilisieren der Einschlusskörper umfasst.

22. Verfahren nach Anspruch 21, wobei das Solubilisieren durch Verwendung eines Mittels zum Solubilisieren durchgeführt wird, das aus der Gruppe ausgewählt ist, die aus Harnstoff in nicht denaturierenden Konzentrationen (1-2 M), N-Laurylsarcosin in nicht denaturierenden Konzentrationen (0,05-0,25 % (g/v)), zwitterionischen Detergentien in niedrigen, nicht denaturierenden Konzentrationen, Nicht-Detergens-Sulfobetainen (NDSBs), Betain, Sarcosin, Carbamoylsarcosin, Taurin, DMSO und einem Puffer in einer hohen solubilisierenden Konzentration, wobei der Puffer aus der Gruppe ausgewählt ist, die aus HEPES, HEPPS, MES, ACES und MES besteht, besteht.

23. Verfahren nach Anspruch 22, wobei das ausgewählte Lösemittel N-Lauroylsarcosin ist.

24. Verfahren nach Anspruch 23, wobei die Konzentration von N-Lauryisarcosin in einem Bereich von 0,1 bis 0,25 % liegt.

25. Verfahren nach Anspruch 24, wobei die Konzentration von N-Laurylsarcosin etwa 0,2 % beträgt.

26. Verfahren zur Herstellung von biologisch aktivem G-CSF durch Biosynthese in E. coli, wobei die Parameter für den Weg der Durchführung der Biosynthese wie folgt ausgewählt sind:
- Kultivierungstemperatur: 20-30 °C, vorzugsweise etwa 25 °C;
- Zusammensetzung des Kultivierungsmediums: ausgewählt aus der Gruppe, die aus GYST, GYSP, LYSP, LYST, LBON und GYSPON, vorzugsweise GYST und GYSP, besteht;
- Typ der Fermentation: Batch-Modus;
- Induktionsmodus: Zugabe von IPTG, im Fall einer optionalen Vorkultivierungsstufe nach der Vorkultivierungsstufe, zu Beginn der Fermentation zur Einstellung einer End-IPTG-Konzentration in einem Bereich von 0,3 bis 0,6 mM, vorzugsweise auf etwa 0,4 mM.

27. Verfahren zur Herstellung von biologisch aktivem G-CSF nach Anspruch 26, wobei das Verfahren ferner ein Isolieren der Einschlusskörper und ein Waschen der isolierten Einschlusskörper mit Wasser umfasst.

28. Verfahren zur Herstellung von biologisch aktivem G-CSF nach Anspruch 26 oder 27, wobei das Verfahren ferner das Solubilisieren der Einschlusskörper mit N-Laurylsarcosin mit einer Konzentration von 0,1 bis 0,25 %, vorzugsweise von etwa 0,2 % umfasst.

29. Verfahren nach einem der Ansprüche 1 bis 28, das die weitere Stufe der Verwendung des G-CSF bei der Herstellung eines Medikaments umfasst.

## Revendications

1. Procédé de production du G-CSF qui implique l'expression dudit G-CSF en tant que protéine hétérologue dans *E. coli,* **caractérisé en ce que** le précurseur correctement plié du G-CSF suite à l'expression s'accumule dans les corps d'inclusion, **caractérisé en ce que** la réalisation de la biosynthèse comprend l'ajustement de la température de culture entre 20°C et 30°C, et **en ce que** la réalisation de la biosynthèse comprend l'ajustement du mode d'induction qui comprend le choix de l'inducteur à partir du groupe composé : (a) de l'IPTG selon une concentration comprise dans la gamme allant de 0,1 mM à 1 mM, (b) de lactose selon une concentration comprise dans la gamme entre 1 et 10 g/l, et (c) de NaCl selon une concentration comprise dans la gamme allant de 0,3 M à 1,3 M.

2. Procédé selon la revendication 1, **caractérisé en ce que** le procédé implique la réalisation de la biosynthèse du G-CSF qui comprend l'ajustement d'un ou deux paramètres qui sont sélectionnés à partir du groupe composé de la composition du milieu de culture, du mode d'induction, du principe de réalisation de la fermentation, de l'ajout d'un agent capable de provoquer une pression et de la co-expression de protéines auxiliaires.

3. Procédé selon l'une quelconque des revendications 1 ou 2, **caractérisé en ce que** la température de culture est d'environ 25°C.

4. Procédé selon l'une quelconque des revendications 1 à 3, **caractérisé en ce que** l'inducteur sélectionné est l'IPTG.

5. Procédé selon la revendication 4, **caractérisé en ce que** la concentration d'IPTG est comprise dans la gamme allant de 0,3 mM à 0,6 mM.

6. Procédé selon la revendication 4, **caractérisé en ce que** la concentration d'IPTG est d'environ 0,4 mM.

7. Procédé selon la revendication 4, **caractérisé en ce que** la concentration d'IPTG est d'environ 1 mM.

8. Procédé selon l'une quelconque des revendications 1 à 3, **caractérisé en ce que** l'inducteur sélectionné est le lactose et **en ce que** la concentration de lactose est comprise entre 2 et 4 g/l.

9. Procédé selon la revendication 8, **caractérisé en ce que** l'inducteur sélectionné est le NaCl et **en ce que** la concentration de NaCl est comprise dans la gamme allant de 1,0 à 1,3 M.

10. Procédé selon l'une quelconque des revendications 1 à 9, **caractérisé en ce que** l'ajustement du mode d'induction comprend l'ajout de l'inducteur au début de la fermentation.

11. Procédé selon l'une quelconque des revendications 1 à 10, **caractérisé en ce que** l'inducteur est ajouté instantanément suite à l'étape de pré-culture, si une étape de pré-culture est mise en place.

12. Procédé selon l'une quelconque des revendications 1 à 11, **caractérisé en ce que** le principe de réalisation de la biosynthèse est sélectionné à partir du groupe composé de la réalisation de la fermentation selon un mode de traitement différé, de la réalisation de la fermentation selon un mode discontinu et de la fermentation dans des flacons sous agitation.

13. Procédé selon la revendication 12, **caractérisé en ce que** le principe de réalisation sélectionné est la fermentation selon un mode de traitement différé.

14. Procédé selon l'une quelconque des revendications 1 à 13, **caractérisé en ce que** le milieu est sélectionné à partir du groupe composé de : GYST, GYSP, LYSP, LYST, LBON et GYSPON.

15. Procédé selon la revendication 14, **caractérisé en ce que** le milieu sélectionné est GYST ou GYSP.

16. Procédé selon l'une quelconque des revendications 2 à 15, **caractérisé en ce que** l'additif qui est capable de provoquer une pression est sélectionné à partir du groupe composé d'éthanol et de propanol.

17. Procédé selon l'une quelconque des revendications 1 à 16 comprenant également le lavage des corps d'inclusion.

18. Procédé selon la revendication 17, **caractérisé en ce que** le lavage est réalisé à l'aide d'une solution qui est sélectionnée à partir du groupe composé d'un tampon Tris/HCl, d'un tampon phosphate, d'un tampon acétate, d'un tampon citrate et d'eau.

19. Procédé selon la revendication 18, **caractérisé en ce que** la concentration du tampon sélectionné est comprise dans la gamme allant de 1 mM à 10 mM.

20. Procédé selon la revendication 18, **caractérisé en ce que** la solution sélectionnée est de l'eau.

21. Procédé de production d'une protéine selon l'une quelconque des revendications 1 à 20, **caractérisé en ce que** le procédé comprend également la solubilisation des corps d'inclusion.

22. Procédé selon la revendication 21, **caractérisé en ce que** la solubilisation est réalisée à l'aide d'un agent de solubilisation qui est sélectionné à partir du groupe composé : d'urée selon des concentrations non-dénaturantes (1-2 M), de N-lauryl sarcosine selon des concentrations non-dénaturantes (0,05-0,25% (m/v)), de Zwitter agents selon des concentrations faibles, non-dénaturantes, de sulfobétaïnes non-détergeantes (NDSB), de bétaïne, de sarcosine, de carbamoyl sarcosine, de taurine, de DMSO et d'un tampon selon une importante concentration de solubilisation, **caractérisé en ce que** le tampon est sélectionné à partir du groupe composé de : HEPES, HEPPS, MES, ACES, et MES.

23. Procédé selon la revendication 22, **caractérisé en ce que** le solvant sélectionné est la N-lauroyl-sarcosine.

24. Procédé selon la revendication 23, **caractérisé en ce que** la concentration de N-lauryl sarcosine est comprise dans la gamme allant de 0, 1 % à 0,25%.

25. Procédé selon la revendication 24, **caractérisé en ce que** la concentration de N-lauryl sarcosine est d'environ 0,2%.

26. Procédé de production du G-CSF biologiquement actif par biosynthèse dans *E. coli,* **caractérisé en ce que** les paramètres pour la réalisation de la biosynthèse sont sélectionnés de la façon suivante :
• température de culture : 20-30°C, de préférence environ 25°C ;
• composition du milieu de culture : sélectionné à partir du groupe composé de GYST, GYSP, LYSP, LYST, LBON et GYSPON, de préférence GYST et GYSP.
• type de fermentation : mode différé
• mode d'induction : ajout d'IPTG, dans le cas d'une éventuelle étape de pré-culture après ladite étape de pré-culture, au début de la fermentation, de façon à ajuster une concentration finale en IPTG dans la gamme allant de 0,3 à 0,6 mM, de préférence, d'environ 0, 4 mM.

27. Procédé de production du G-CSF biologiquement actif selon la revendication 26, **caractérisé en ce que** le procédé comprend également l'isolation des corps d'inclusion et le lavage des corps d'inclusion isolés avec de l'eau.

28. Procédé de production du G-CSF biologiquement actif selon la revendication 27 ou 28, **caractérisé en ce que** le procédé comprend également la solubilisation des corps d'inclusion avec de la N-lauryl sarcosine selon une concentration comprise dans la gamme allant de 0,1 à 0,25%, de préférence d'environ 0,2%.

29. Procédé selon l'une quelconque des revendications 1 à 28, comprenant l'étape supplémentaire de fusion du G-CSF pour la préparation d'un médicament.
